# EUROPEAN PATENT APPLICATION

(11) **EP 4 191 267 A1**
(43) Date of publication of application: **07.06.2023**
(21) Application number: 22164389.3
(22) Date of filing: 25.03.2022
(51) Int. Cl.: G01R 33/58, G01R 33/28, G01R 33/54, A61B 6/00, A61B 8/00, A61N 5/10, G01R 33/48

(54) **METHOD AND SYSTEM FOR SEMI-AUTOMATED QUALITY (QA) ASSURANCE APPROACH FOR MEDICAL SYSTEMS**

(30) Priority: 02.12.2021 US 202163285248 P
(71) Applicant: Koninklijke Philips N.V., 5656 AG Eindhoven (NL)
(72) Inventor: SOSSIN, Artur, Eindhoven (NL); GRAESSLIN, Ingmar, Eindhoven (NL)
(74) Representative: Philips Intellectual Property & Standards

(57) **Abstract**

A non-transitory computer readable medium **(26s)** stores instructions executable by at least one electronic processor **(14s)** to perform a method **(100)** of monitoring a quality assurance (QA) procedure performed using a medical device **(2).** The method includes receiving a signal indicating a start of the QA procedure; analyzing video **(17)** of the medical device acquired after receiving the signal to detect one or more errors during the QA procedure; and providing a remedial action addressing the detected one or more errors.

## Description

### FIELD OF THE INVENTION

The following relates generally to the imaging arts, remote imaging assistance arts, remote imaging examination monitoring arts, quality assurance procedure arts, and related arts.

### BACKGROUND OF THE INVENTION

Regular quality assurance (QA) of a medical imaging system ensures consistent diagnostic image quality. Depending on the modality and the image quality assurance standards (QAS) adopted by a given healthcare provider, QA procedures can be performed on daily, weekly, or monthly bases with varying degrees of complexity. Depending on the level of complexity, medical imaging system QA is often time consuming and can require a set of specialized materials and tools (software/hardware). Furthermore, to perform these procedures, trained staff, such as a medical physicist or a corresponding service engineer, can also be required.

A typical QA procedure used for assessing a medical imaging system may employ a selected one of a set of standardized quality phantoms, and tools to support the measurements. The quality tests are then run by a service engineer (on site or remotely) together with a radiology technologist (RT). The data acquired during such scans is processed on the machine and a report is be generated to detect any irregularities. More complex QA tasks, and QA tasks for certain modalities, may involve the physical presence of either a service engineer (SE) or medical physicist on site. It typically is not feasible for the manufacturer to send service engineers to oversee routine daily or weekly QA routines. Furthermore, in the case of an SE supporting the RT remotely, the lack of visual information and more comprehensive verification of phantom or tool positioning can lead to reduced measurement quality for QA tasks overseen remotely by the SE.

The following discloses certain improvements to overcome these problems and others.

### SUMMARY OF THE INVENTION

In one aspect, a non-transitory computer readable medium stores instructions executable by at least one electronic processor to perform a method of monitoring a quality assurance (QA) procedure performed using a medical device. The method includes receiving a signal indicating a start of the QA procedure; analyzing video of the medical device acquired after receiving the signal to detect one or more errors during the QA procedure; and providing a remedial action addressing the detected one or more errors.

In another aspect, an apparatus includes a medical imaging device; and instructions stored in a non-transitory computer readable medium executable by at least one electronic processor to perform a method of monitoring a QA procedure performed using the medical imaging device. The method includes receiving an image acquired by the medical imaging device during the QA procedure; analyzing the received image to identify a deficiency of the medical imaging device; determining an adjustment to the medical imaging device for resolving the identified deficiency of the medical imaging device; and proposing the adjustment via a display device.

In another aspect, a method of monitoring a QA procedure performed using a medical device includes receiving a signal indicating a start of the QA procedure; analyzing video of the medical device acquired after receiving the signal to detect one or more errors during the QA procedure; providing a remedial action addressing the detected one or more errors; displaying the video of the medical device on a remote expert workstation located remotely from the medical device; and providing two-way communication between the remote expert workstation and an electronic device disposed with the medical device to perform the remedial action.

One advantage resides in providing for a QA procedure with support from a remote expert to a local operator performing the QA procedure.

Another advantage resides in providing for a QA procedure for a medical imaging device with assistance in aspects such as selection and placement of an imaging phantom.

Another advantage resides in providing for a QA procedure with immediate analysis of QA images and actionable guidance for improving imaging quality based on the analysis.

Another advantage resides in reduced errors, repeats, and reduced wasted time for medical procedures.

A given embodiment may provide none, one, two, more, or all of the foregoing advantages, and/or may provide other advantages as will become apparent to one of ordinary skill in the art upon reading and understanding the present disclosure.

### BRIEF DESCRIPTION OF THE DRAWINGS

The example embodiments are best understood from the following detailed description when read with the accompanying drawing figures. It is emphasized that the various features are not necessarily drawn to scale. In fact, the dimensions may be arbitrarily increased or decreased for clarity of discussion. Wherever applicable and practical, like reference numerals refer to like elements.
Fig. 1 diagrammatically shows an illustrative apparatus for providing remote assistance in accordance with the present disclosure.

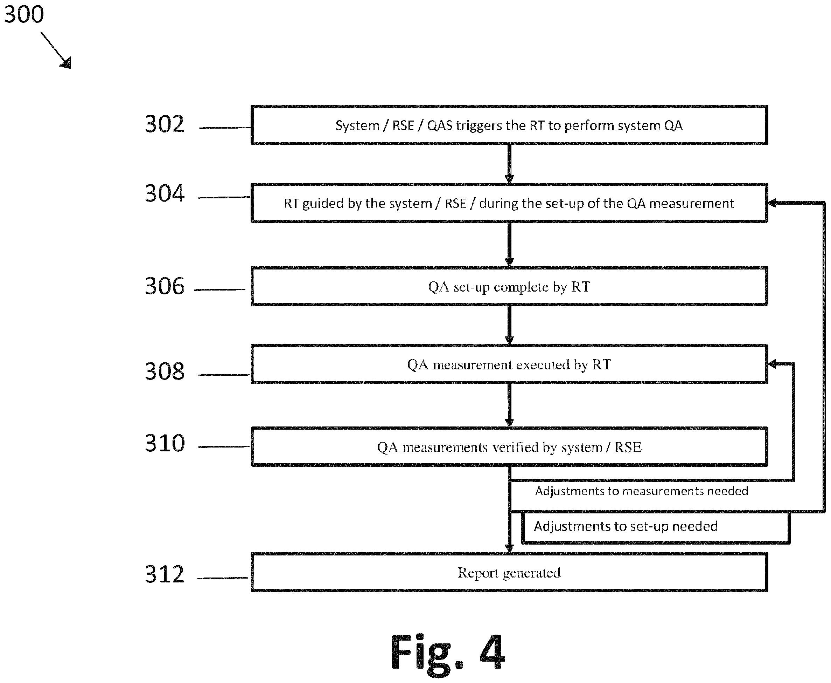

Figs.2-4 show example flow charts of operations suitably performed by the apparatus of Fig. 1.

### DETAILED DESCRIPTION OF EMBODIMENTS

The following is motivated in part by the recognition made herein, based on feedback obtained from medical imaging device service engineers, which has identified problems with quality assurance (QA) procedures as a prominent source of unnecessary product servicing and component returns. In a typical QA procedure, an object, such as an imaging phantom, is used as a test subject, and imaging system performance is assessed based on acquired images of the phantom. In practice, it appears that customers (e.g., hospital radiology departments and other medical imaging device operators) are commonly performing QA procedures with the phantom and/or auxiliary components such as MR imaging coils misplaced, or even using the wrong phantom. These mistakes in performing QA procedures can lead to unnecessary service calls and reduced customer satisfaction.

Disclosed herein are approaches for remotely supervising QA procedures. A camera is placed in the imaging bay, and possibly a second camera may be arranged to image the examination region (e.g., inside the bore of an MRI or CT). The camera provides real time visual monitoring of a QA procedure in progress.

The disclosed approaches advantageously leverage infrastructure that may already be present in the context of a Radiology operations command center (ROCC) or similar imaging assistance system, which provides infrastructure for imaging centers or integrated delivery networks (IDNs) to interact with pools of technologist ("tech"; also referred to herein as "radiation technologist" or "RT") talent to share tech expertise across an entire imaging network. By providing a communication channel (e.g., telephonic, videoconferencing, or so forth) and remote imaging device controller console sharing, an ROCC empowers more experienced techs to provide guidance and oversight for junior techs when working with an imaging modality or workflow they may not be familiar or comfortable with. The ROCC infrastructure may include a bay camera and possibly also a "bore" camera arranged to image the examination region, and these can be repurposed as disclosed herein to provide automated or semi-automated QA support.

The QA procedure supervision may be provided by a remote service engineer (RSE), or may be provided in an automated fashion using a supervisory script or artificial intelligence (AI) component. Feedback from the RSE or AI supervisor to the on-site radiology technologist (RT) performing the QA procedure can be provided via an application program running on a computer or mobile device, or in the case of RSE supervision by telephonic or videoconference link.

Various approaches can be used to detect errors in the QA procedure in progress based on the video feedback. The phantom can be identified using a suitable object recognition component, such as a convolutional neural network (CNN) trained to recognize the correct phantom versus other likely incorrect phantoms (e.g., a different one from another manufacturer or that is used in a different type of QA procedure). To detect mispositioning of the phantom or other component, if a light visor is used for aligning the phantom or other component then the camera can directly observe any displacement between the phantom or other component and the alignment mark(s) provided by the light visor. If the camera is a three-dimensional (3D) camera then this is straightforward. If the camera is a two-dimensional (2D) camera, then a suitable algorithm can be used to estimate 3D position from the 2D camera image, Such algorithms can include, for example, deep learning methods to estimate the depth information including a distance from camera to each object point and therefore reconstruct the 3D position of the object (see, e.g., e.g. PlaneNet: Piece-wise Planar Reconstruction from a Single RGB Image et al 2018), using geometrical relationships between 3D world coordinates and 2D camera image coordinates using known object dimensions, known camera properties (e.g., intrinsic parameter, extrinsic parameters, location, etc.) and the detected object in the 2D image (see, e.g., Bradski, G., and A. Kaehler. Learning OpenCV: Computer Vision with the OpenCV Library. Sebastopol, CA: O'Reilly, 2008).

A common problem in QA procedures is misorientation or (in the case of deformable phantoms) incorrect shaping of the phantom. This can be detected using a CNN or other machine learning component in a fashion similar to the object recognition component.

Various remedial actions can be taken. In one approach, the RSE or automated script or AI supervisor provides verbal or visual feedback indicating a wrong phantom or misalignment, or other detected errors in the QA procedure in progress. In the case of a wrong phantom or misshapen deformable phantom, a display can show the correct phantom/shape.

In some embodiments disclosed herein, a projector is integrated with the imaging device to provide the feedback. For example, a projector could project an image of the correct phantom and its correct position on the table. A holographic projector could even show this in 3D.

In other embodiments disclosed herein, proactive remediation of mistakes being made during the QA procedure is also contemplated. For example, if the table has automated positioning that is remotely controllable by the script or AI supervisor then this can be used to automatically correct translational mispositioning on the table so that the phantom is positioned at scanner isocenter (i.e., center of the field-of-view) during the QA procedure. In the case of MRI, the magnetic field gradients defining imaging isocenter could be adjusted to provide analogous translational correction by moving the isocenter to coincide with the position of the imaging phantom.

In another embodiment, an augmented reality (AR) headset or AR eyeglasses could be used to provide the feedback as superimposed AR content. A forward-looking camera of the AR headset or glasses could also augment or be substituted for the bay and/or bore camera(s) to provide video of the QA procedure.

The disclosed system is also contemplated to provide various validation aspects based on the images acquired by the imaging device during the QA procedure. For example, an image feature that can be linked to an *a priori* known underlying cause can be recognized by analysis of the QA procedure images and the RT advised to check for that possible underlying cause. As a specific example, the system may guide the RT through an imaging device adjustment process, acquiring further QA procedure images, and so forth until the issue is resolved.

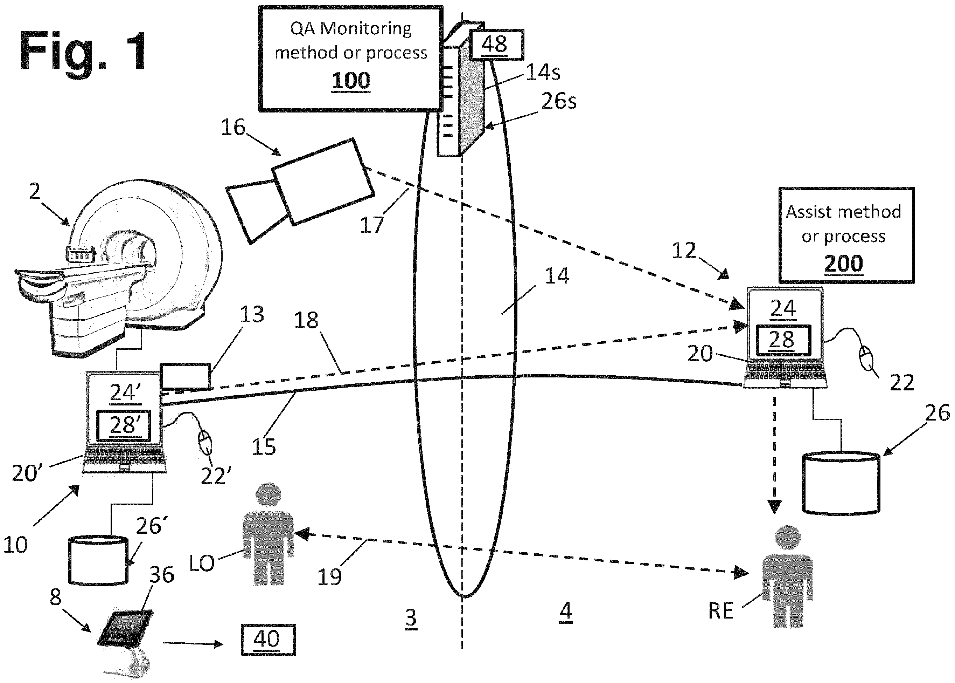

With reference to FIGURE 1, an apparatus for providing assistance from a remote medical imaging expert **RE** (or supertech such as an experienced technologist) to a local radiologist technician or local technician operator **LO** is shown. Such a system is also referred to herein as a radiology operations command center (ROCC). As shown in Fig. 1, the local operator **LO**, who operates a medical imaging device (also referred to as an image acquisition device, imaging device, and so forth) **2**, is located in a medical imaging device bay 3, and the remote expert **RE** is disposed in a remote service location or center **4.** It should be noted that the remote expert **RE** may not necessarily directly operate the medical imaging device **2**, but rather provides assistance to the local operator **LO** in the form of advice, guidance, instructions, or the like. The remote location **4** can be a remote service center, a radiologist's office, a radiology department, and so forth. The remote location **4** may be in the same building as the medical imaging device bay **3** (this may, for example, in the case of a remote expert **RE** who is a radiologist tasked with peri-examination image review), or the remote service center **4** and the medical imaging device bay **3** may be in different buildings, and indeed may be located in different cities, different countries, and/or different continents. In general, the remote location **4** is remote from the imaging device bay **3** in the sense that the remote expert **RE** cannot directly visually observe the imaging device **2** in the imaging device bay **3** (hence optionally providing a video feed as described further herein).

The image acquisition device 2 can be a Magnetic Resonance (MR) image acquisition device, a Computed Tomography (CT) image acquisition device; a positron emission tomography (PET) image acquisition device; a single photon emission computed tomography (SPECT) image acquisition device; an X-ray image acquisition device; an ultrasound (US) image acquisition device; or a medical imaging device of another modality. The imaging device 2 may also be a hybrid imaging device such as a PET/CT or SPECT/CT imaging system. While a single image acquisition device 2 is shown by way of illustration in Fig. 1, more typically a medical imaging laboratory will have multiple image acquisition devices, which may be of the same and/or different imaging modalities. For example, if a hospital performs many CT imaging examinations and relatively fewer MRI examinations and still fewer PET examinations, then the hospital's imaging laboratory (sometimes called the "radiology lab" or some other similar nomenclature) may have three CT scanners, two MRI scanners, and only a single PET scanner. This is merely an example. Moreover, the remote service center **4** may provide service to multiple hospitals. The local operator **LO** controls the medical imaging device **2** via an imaging device controller **10.** The remote expert **RE** is stationed at a remote workstation **12** (or, more generally, an electronic controller 12).

As used herein, the term "medical imaging device bay" (and variants thereof) refer to a room containing the medical imaging device **2** and also any adjacent control room containing the medical imaging device controller **10** for controlling the medical imaging device. For example, in reference to an MRI device, the medical imaging device bay **3** can include the radiofrequency (RF) shielded room containing the MRI device **2**, as well as an adjacent control room housing the medical imaging device controller **10**, as understood in the art of MRI devices and procedures. On the other hand, for other imaging modalities such as ultrasound (US) imaging, the imaging device controller **10** may be located in the same room as the imaging device **2**, so that there is no adjacent control room and the medical bay **3** is only the room containing the medical imaging device **2.** The imaging device controller **10** includes an electronic processor **20'**, at least one user input device such as a mouse **22'**, a keyboard, and/or so forth, and a display device **24'.** The imaging device controller **10** presents a device controller graphical user interface (GUI) **28'** on the display **24'** of the imaging device controller **10**, via which the local operator **LO** accesses device controller GUI screens for entering the imaging examination information such as the name of the local operator **LO**, the name of the patient and other relevant patient information (e.g. gender, age, etc.) and for controlling the (typically robotic) patient support to load the patient into the bore or imaging examination region of the imaging device **2**, selecting and configuring the imaging sequence(s) to be performed, acquiring preview scans to verify positioning of the patient, executing the selected and configured imaging sequences to acquire clinical images, display the acquired clinical images for review, and ultimately store the final clinical images to a Picture Archiving and Communication System (PACS) or other imaging examinations database. In addition, while FIGURE 1 shows a single medical imaging device bay **3**, it will be appreciated that the remote service center **4** (and more particularly the remote workstation **12)** is in communication with multiple medical bays via a communication link **14**, which typically comprises the Internet augmented by local area networks at the remote operator **RE** and local operator **LO** ends for electronic data communications.

As diagrammatically shown in Fig. 1, in some embodiments, a camera **16** (e.g., a video camera) is arranged to acquire a video stream **17** of a portion of the medical imaging device bay **3** that includes at least the area of the imaging device **2** where the local operator **LO** interacts with the patient, and optionally may further include the imaging device controller **10.** The video stream **17** is sent to the remote workstation **12** via the communication link **14**, e.g., as a streaming video feed received via a secure Internet link. In some examples, as shown in Fig.1, the camera **16** can be affixed to a wall of ceiling of the medical facility with a field of view to include the area of the imaging device **2** where the local operator **LO** interacts with the patient, and optionally may further include the imaging device controller **10.** In other examples, the camera **16** can be disposed within an imaging bore (not shown) of the imaging device **2.**

In other embodiments, the live video feed **17** of the display **24'** of the imaging device controller **10** is, in the illustrative embodiment, provided by a video cable splitter **15** (e.g., a DVI splitter, a HDMI splitter, and so forth). In other embodiments, the live video feed **17** may be provided by a video cable connecting an auxiliary video output (e.g. aux vid out) port of the imaging device controller **10** to the remote workstation **12** operated by the remote expert **RE.** Alternatively, a screen mirroring data stream **18** is generated by screen sharing software **13** running on the imaging device controller **10** which captures a real-time copy of the display **24'** of the imaging device controller **10**, and this copy is sent from the imaging device controller **10** to the remote workstation **12.** Other approaches besides the illustrative video cable splitter **15** or screen sharing software **13** are contemplated for capturing a real-time copy of the display **24'** of the imaging device controller **10** which is then sent to the workstation **12** of the remote expert **RE.** While in an ROCC this real-time copy of the display **24'** of the imaging device controller **10** is used to provide status information to the remote expert **RE** for use in assisting the local operator **LO**, in embodiments disclosed herein the real-time copy of the display **24'** of the imaging device controller **10** is also leveraged (optionally along with other available information) to determine one or more performance metrics of the local operator **LO.**

The communication link **14** also provides a natural language communication pathway **19** for verbal and/or textual communication between the local operator **LO** and the remote expert **RE**, in order to enable the latter to assist the former in performing the imaging examination. For example, the natural language communication link **19** may be a Voice-Over-Internet-Protocol (VOIP) telephonic connection, a videoconferencing service, an online video chat link, a computerized instant messaging service, or so forth. Alternatively, the natural language communication pathway **19** may be provided by a dedicated communication link that is separate from the communication link **14** providing the data communications **17**, **18**, e.g., the natural language communication pathway **19** may be provided via a landline telephone. In another example, the natural language communication pathway **19** may be provided via an ROCC device **8**, such as a mobile device (e.g., a tablet computer or a smartphone), or can be a wearable device worn by the local operator **LO**, such as an augmented reality (AR) display device (e.g., AR goggles), a projector device, a heads-up display (HUD) device, etc., each of which having a display device **36.** For example, an "app" can run on the ROCC device **8** (operable by the local operator **LO)** and the remote workstation **12** (operable by the remote expert **RE)** to allow communication (e.g., audio chats, video chats, and so forth) between the local operator and the remote expert.

Fig.1 also shows, in the remote service center **4** including the remote workstation **12**, such as an electronic processing device, a workstation computer, or more generally a computer, which is operatively connected to receive and present the video **17** of the medical imaging device bay **3** from the camera **16** and to present the screen mirroring data stream **18** as a mirrored screen. Additionally or alternatively, the remote workstation **12** can be embodied as a server computer or a plurality of server computers, e.g., interconnected to form a server cluster, cloud computing resource, or so forth. The workstation **12** includes typical components, such as an electronic processor **20** (e.g., a microprocessor), at least one user input device (e.g., a mouse, a keyboard, a trackball, and/or the like) **22**, and at least one display device **24** (e.g., an LCD display, plasma display, cathode ray tube display, and/or so forth). In some embodiments, the display device **24** can be a separate component from the workstation **12.** The display device **24** may also comprise two or more display devices, e.g., one display presenting the video **17** and the other display presenting the shared screen (i.e., display **24')** of the imaging device controller **10** generated from the screen mirroring data stream **18.** Alternatively, the video and the shared screen may be presented on a single display in respective windows. The electronic processor **20** is operatively connected with a one or more non-transitory storage media **26.** The non-transitory storage media **26** may, by way of non-limiting illustrative example, include one or more of a magnetic disk, RAID, or other magnetic storage medium; a solid-state drive, flash drive, electronically erasable read-only memory (EEROM) or other electronic memory; an optical disk or other optical storage; various combinations thereof; or so forth; and may be for example a network storage, an internal hard drive of the workstation **12**, various combinations thereof, or so forth. It is to be understood that any reference to a non-transitory medium or media **26** herein is to be broadly construed as encompassing a single medium or multiple media of the same or different types. Likewise, the electronic processor **20** may be embodied as a single electronic processor or as two or more electronic processors. The non-transitory storage media **26** stores instructions executable by the at least one electronic processor **20.** The instructions include instructions to generate a graphical user interface (GUI) **28** for display on the remote operator display device **24.**

The medical imaging device controller **10** in the medical imaging device bay **3** also includes similar components as the remote workstation **12** disposed in the remote service center **4.** Except as otherwise indicated herein, features of the medical imaging device controller **10** disposed in the medical imaging device bay **3** similar to those of the remote workstation **12** disposed in the remote service center **4** have a common reference number followed by a "prime" symbol (e.g., processor **20'**, display **24'**, GUI **28')** as already described. In particular, the medical imaging device controller **10** is configured to display the imaging device controller GUI **28'** on a display device or controller display **24'** that presents information pertaining to the control of the medical imaging device **2** as already described, such as imaging acquisition monitoring information, presentation of acquired medical images, and so forth. It will be appreciated that the real-time copy of the display **24'** of the controller **10** provided by the video cable splitter **15** or the screen mirroring data stream **18** carries the content presented on the display device **24'** of the medical imaging device controller **10.** The communication link **14** allows for screen sharing from the display device **24'** in the medical imaging device bay **3** to the display device **24** in the remote service center **4.** The GUI **28'** includes one or more dialog screens, including, for example, an examination/scan selection dialog screen, a scan settings dialog screen, an acquisition monitoring dialog screen, among others. The GUI **28'** can be included in the video feed **17** or provided by the video cable splitter **15** or by the mirroring data stream **17'** and displayed on the remote workstation display **24** at the remote location **4.**

Fig.1 shows an illustrative local operator **LO**, and an illustrative remote expert **RE** (i.e., expert, e.g., supertech). However, the ROCC optionally provides a staff of supertechs who are available to assist local operators **LO** at different hospitals, radiology labs, or the like. The ROCC may be housed in a single physical location or may be geographically distributed. For example, in one contemplated implementation, the remote operators **RO** are recruited from across the United States and/or internationally in order to provide a staff of supertechs with a wide range of expertise in various imaging modalities and in various imaging procedures targeting various imaged anatomies. In view of this multiplicity of local operators **LO** and multiplicity of remote operators **RO**, the disclosed communication link **14** includes a server computer **14s** (or a cluster of servers, cloud computing resource comprising servers, or so forth) which is programmed to establish connections between selected local operator **LO**/remote expert **RE** pairs. For example, if the server computer **14s** is Internet-based, then connecting a specific selected local operator **LO**/remote expert **RE** pair can be done using Internet Protocol (IP) addresses of the various components **16**, **10**, **12**, the telephonic or video terminals of the natural language communication pathway **19**, et cetera. The server computer **14s** is operatively connected with a one or more non-transitory storage media **26s.** The non-transitory storage media **26s** may, by way of non-limiting illustrative example, include one or more of a magnetic disk, RAID, or other magnetic storage medium; a solid state drive, flash drive, electronically erasable read-only memory (EEROM) or other electronic memory; an optical disk or other optical storage; various combinations thereof; or so forth; and may be for example a network storage, an internal hard drive of the server computer **14s**, various combinations thereof, or so forth. It is to be understood that any reference to a non-transitory medium or media **26s** herein is to be broadly construed as encompassing a single medium or multiple media of the same or different types. Likewise, the server computer **14s** may be embodied as a single electronic processor or as two or more electronic processors. The non-transitory storage media **26s** stores instructions executable by the server computer **14s.** In addition, the non-transitory computer readable medium **26s** (or another database) stores data related to a set of remote experts **RE** and/or a set of local operators **LO.** The remote expert data can include, for example, skill set data, work experience data, data related to ability to work on multi-vendor modalities, data related to experience with the local operator **LO** and so forth.

Furthermore, as disclosed herein the server **14s** performs a method or process **100** of monitoring a quality assurance (QA) procedure performed using the medical device **2.** The QA procedure monitoring method **100** advantageously leverages information sources provided by the ROCC, such as the content of the display **24'** of the imaging device controller **10.**

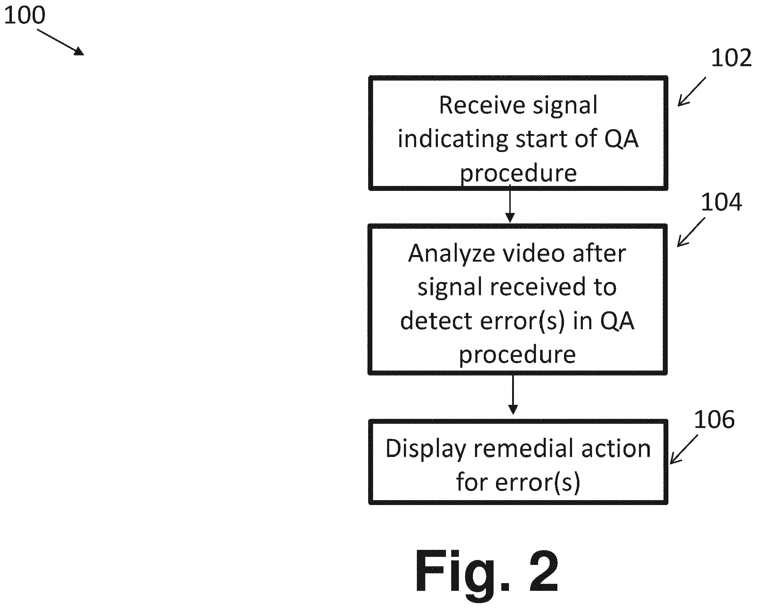

With reference to Fig.2, and with continuing reference to Fig.1, an illustrative embodiment of the QA procedure monitoring method **100** is diagrammatically shown as a flowchart. At an operation **102**, the remote expert **RE** receives a signal on the remote workstation **12** that indicates a start of a QA procedure. This signal may be provided by the local operator **LO** or from the server computer **14s**, or may be automatically generated by the medical imaging device **2** itself when its controller is set up to perform a QA procedure. It should be noted that the remote expert **RE** in the context of supervising a QA procedure might be different from, and may have different expertise than, a remote expert that assists an RT during an imaging examination. For example, the remote expert **RE** supervising a QA procedure may be a remote service engineer (RSE) with engineering expertise in the imaging device *per se* but possibly without medical expertise; whereas the remote expert providing assistance to the RT during an imaging examination may be a trained radiology technician with medical expertise but possibly without engineering expertise. As another example, the **RE** supervising a QA procedure may be an artificial intelligence (AI) supervisor or a script specifically programmed for providing the QA procedure supervision. Moreover, while the illustrative embodiment includes both ROCC and QA supervisory capability, thus advantageously leveraging the ROCC hardware such as camera 16 for QA supervision, in other embodiments the QA supervision may be provided without ROCC capability. In this latter case, the hardware such camera **16** would be installed specifically for QA supervision (or possibly for some other purpose such as security monitoring).

With continuing reference to Fig.2, at an operation **104**, the video stream **17** (and/or the audio stream **18)** acquired after the signal is received is analyzed to detect one or more errors during the QA procedure. The analysis operation **104** can be performed in a variety of manners. In one embodiment, the video stream **17** is analyzed to detect a sequence of events of the QA procedure, and an error is detected during the QA procedure by comparing the detected sequence of events with a workflow of the QA procedure (stored in the non-transitory computer readable medium **26s** of the server computer **14s).** In another embodiment, the video stream **17** is analyzed to detect an object being used in the QA procedure that is different from a correct object for the QA procedure. In another embodiment, the video stream **17** is analyzed to detect an incorrect position of an object used in the QA procedure. For example, when the medical device **2** comprises a medical imaging device, the object can be an imaging phantom used in the QA procedure. In another example, when the medical device **2** comprises an MRI scanner, the object can be a local coil used in the QA procedure. In any of these examples, the analyzing operation **104** can include inputting the video stream **17** to an artificial neural network (ANN) **48** (implemented in the server computer **14s)** applied to one or more frames of the video stream **17.** For example, the ANN **48** can comprise a convolutional NN (CNN) **48.** In some embodiments, the detected error(s) (or points of help in the QA procedure) can be used for future educational content assessment materials (i.e., training videos or modules) for individuals, or for departments at a medical facility. In other embodiments, the detected one or more errors can be logged within service logs (or separately from service logs) and stored in the non-transitory computer readable medium **26s** of the server computer **14s**, and used in future identification of errors/and roots cause analysis of issues or failures of the medical device **2.**

At an operation **106**, a remedial action addressing the detected one or more errors is provided. The remedial action operation **106** can also be performed in a variety of manners. In one example, a visualization **40** of the remedial action is displayed on the display device **36** of the ROCC device **8** (e.g., regardless of whether the ROCC device **8** comprises a tablet or smartphone, an AR device (so that the AR content of the remedial action is superimposed on the medical device **2)**, a projector, an HUD device, and so forth) so that it is observable by the local operator **LO.** In another example, the remedial action operation **106** can include controlling a position of the medical device **2** (e.g., moving a patient bench of the medical device **2).** In a further example, the remedial action operation **106** can include performing a validation process to determine if the detected one or more errors corresponds to a predetermined matter with the medical device **2.**

In a particular embodiment, the medical device **2** comprises a medical imaging device **2.** In such embodiments, the signal receiving operation **102** includes receiving an image acquired by the medical imaging device during the QA procedure in which the image comprises the signal that indicates a start of a QA procedure. The analysis operation **104** comprises analyzing the received image to identify a deficiency (e.g., an image quality issue) of the medical imaging device **2**, and determining an adjustment to the medical imaging device **2** for resolving the identified deficiency of the medical imaging device **2.**

In one example, the remedial action operation **106** comprises proposing the adjustment via the display device **36** of the ROCC device **8.** In another example, the remedial action operation **106** includes, when the ROCC device **8** is a HUD device **8**, determining a mispositioning of an object based on position of the object compared with alignment marks produced by the projector and observed in the video **17.** In another example, the remedial action operation **106** includes, when the ROCC device **8** is a projector device **8**, projecting a marker indicating a correct placement of an object on an imaging support of the imaging device **2.** In another example, the remedial action operation **106** can include controlling a robotic subject support of the medical imaging device **2** or magnetic field gradient coils of an MRI imaging device **2** to correct a mispositioning of a phantom relative to an isocenter of the imaging device **2.**

Referring back to Fig.1, when the local operator **LO** requires assistance from the remote expert **RE**, the communication link **14** connects the local operator **LO/** remote expert **RE.** The GUI **28** is provided as a remote assistance UI on the display device **24** operable by a remote expert **RE.** The UI **28** provides two-way communication between the local operator **LO** and the remote expert **RE** via which the remote expert can provide assistance to the local medical imaging device operator **LO.**

The remote workstation **12** of the selected remote expert **RE**, and/or the medical imaging device controller **10** being run by the local operator **LO**, is configured to perform a method or process **200** for providing assistance from the remote expert **RE** to the local operator **LO.** For brevity, the method **200** will be described as being performed by the remote workstation **12.** The non-transitory storage medium **26** stores instructions which are readable and executable by the at least one electronic processor **20** (of the workstation **12**, as shown, and/or the electronic processor or processors of a server or servers on a local area network or the Internet) to perform disclosed operations including performing the method or process **200.**

A suitable implementation of the assistance method or process **200** is as follows. The method **200** is performed over the course of (at least a portion of) the QA procedure performed using the medical imaging device **2**, and the local expert **RE** is one selected via the matching method **100.** To perform the method **200**, at an operation **202**, the workstation **12** in the remote location **4** is programmed to receive at least one of: (i) the video **17** from the video camera **16** of the medical imaging device **2** located in the medical imaging device bay **3**; and/or (ii) the screen sharing **18** from the screen sharing software **13**; and/or (iii) the video **17** tapped by the video cable splitter **15.** The video feed **17** and/or the screen sharing **18** can be displayed at the remote workstation display **24**, typically in separate windows of the GUI **28.** At an operation **204**, the video feed **17** and/or the screen sharing **18** can be screen-scraped to determine information related to the medical imaging examination (e.g., modality, vendor, anatomy to be imaged, cause of issue to be resolved, and so forth). In particular, the GUI **28** presented on the display **24** of the remote workstation **12** preferably includes a window presenting the video **17**, and a window presenting the mirrored screen of the medical imaging device controller **10** constructed from the screen mirroring data stream **18**, and status information on the medical imaging examination that is maintained at least in part using the screen-scraped information. This allows the remote operator **RE** to be aware of the content of the display of the medical imaging device controller **10** (via the shared screen) and also to be aware of the physical situation, e.g., position of the patient in the medical imaging device **2** (via the video **17**), and to additionally be aware of the status of the imaging examination as summarized by the status information. In some embodiments, a determination can be made as to whether an imaging procedure using the medical imaging device **2** is a clinical scan, or a QA scan, and can transmit this determination to the remote workstation **12**, and/or can optionally serve as the signal received at operation **102** of Fig.2 to trigger the QA procedure monitoring method **100.** During an imaging procedure, at an operation **206**, the natural language communication pathway **19** is suitably used to allow the local operator **LO** and the remote operator **RE** to discuss the procedure and in particular to allow the remote operator to provide advice to the local operator.

### EXAMPLE

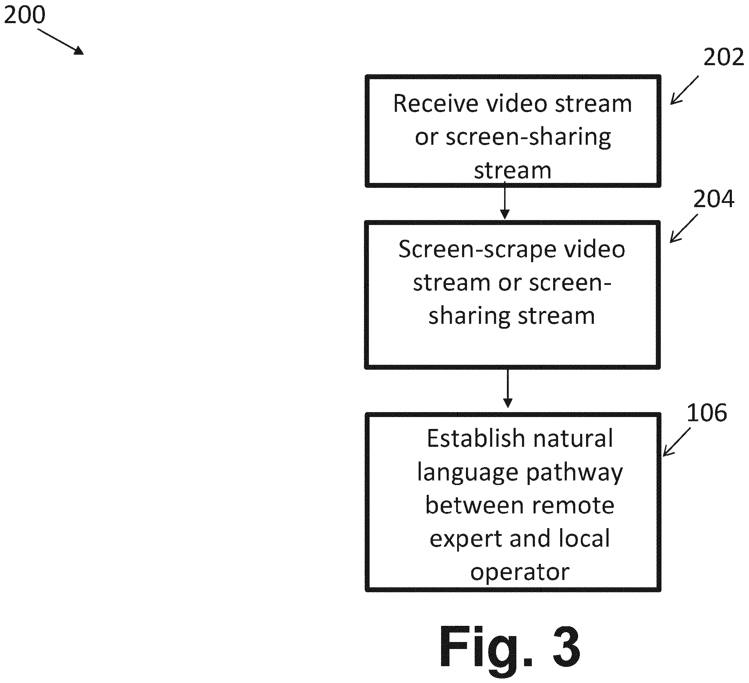

The following describes another example of the method **100.** In order to enable a more simplified workflow during the QA of medical imaging devices **2**, a semi-automated approach comprising several key steps is proposed and is summarized in a method **300** depicted as a flowchart shown in Fig.3. The goal is to reduce the training requirements of staff performing these procedures and improve overall reliability. This enables the manufacturer to provide a more comprehensive service in terms of QA and reduces the need for highly trained personnel like medical physicists on the side of the health care provider.

At an operation **302**, a notification from either the RSE, the system itself or based on the QAS employed in the healthcare institution is provided. Any frequency can be considered, however in the case of involvement of the RSE it may be limited by the service contract. From the manufacturer all the necessary phantom(s) and tool(s) are supplied. If these do not suffice the required QAS of the healthcare provider, additional ones can also be incorporated in the overall approach. In another scenario, the RT starts the QA procedure on his or her own, in which case the triggering operation **302** may be omitted.

At an operation **304**, once the RT is notified with the signal, the RT can configure the medical device **2** (e.g., X-ray tube / detector placement, coil placement, etc.) and correctly place the required items. At some point during either operation **302** or operation **304** the QA procedure supervision is initiated. This can occur by various mechanisms. In one approach, the operation **302** notifies both the RT and the RSE of the start of the QA procedure. In another approach, the operation **302** notifies the RT of the start of the QA procedure and also invokes the AI-based supervision. In another approach, the RT manually calls the RSE or manually starts the AI-based supervision as part of the setup operation **304.** In another approach, if the QA procedure entails bringing up a special QA procedure menu on the controller of the imaging device **2** then the RSE notification or AI-based supervision invocation can be triggered when the special QA procedure menu is brought up. These are merely some illustrative approaches.

At an operation **306**, in one embodiment, the medical bay **3** features the digital camera **16**, either integrated in the medical device **2** or placed externally while allowing sufficient visibility. Throughout this process the RT is guided by the RSE or by the medical device **2** based on visual data coming from the camera **17.** In case of the RSE, audio-visual information **17**, **18** is conveyed to the ROCC device **8** to support the RT. In the case of automatic guidance by the medical device **2**, recognition algorithms are employed on the camera's visual feed to verify the actions of the RT.

In some embodiments, for the QA procedure, quality phantoms need to be positioned in a specific way on the patient table, often requiring special phantom holders. After positioning of the phantom, a light visor is used for alignment and then the phantom is moved into the iso-center of the magnet using the patient table tumble switch. During this QA procedure, the display device **36** of the ROCC device **8** next to a bore of the medical imaging device **2** shows the position overlaid in red onto a table to where the phantom shall then be put. The image stream **17** of the camera **16** is used to detect the phantom on the patient table. To do so, machine-learning (ML) based algorithms (i.e., the CNN **48)** using pre-trained image nets (e.g., U-net) are suitable. The algorithm identifies the phantom (if it is the correct phantom). A notification that the phantom is incorrect is displayed on the ROCC device **8**, or communicated via the natural language pathway **19.** Optionally, the automatically identified imaging phantom may also be highlighted on the ROCC device **8** by a bounding box or the like superimposed on the video display. As another variant, if the incorrect imaging phantom is being used, the ROCC device **8** may display an image of the correct image phantom to assist the RT in selecting the correct phantom.

If the CNN **48** determines that the phantom is the correct one, then the position and orientation of the phantom is determined. Once the phantom is placed on the correct location on the table, the phantom position location color on the ROCC device **8** is switched to green, and optionally a voice command is given to the RT to step back from the patient table and leave the room indicating that the table is now moving to the correct scanning position and once the door is closed the scan starts. Alternatively, if the imaging phantom is incorrectly placed then a valid region for the phantom is shown on the ROCC device **8.** The phantom position is determined by the CNN **48** by evaluating the images and the offset to the correct position in the medical imaging device **2** is calculated and the table is moved automatically into the medical imaging device **2.** If a projector is present, then the appropriate phantom position region is suitably projected on top of the table. Specific phantom holders are sometimes required, but in case it is sufficient to accurately know the position of the phantom it is determined by the CNN **48** and then communicated to the medical imaging device **2** for correct table positioning but also adaptation of the QA scan protocol adjustment with respect to the correct setting of the imaging volume.

In some embodiments, the RT is supplied with the ROCC device **8** as an AR headset or glasses **8** by the manufacturer. The AR glasses allow the RSE or the CNN **48** to access to a visual feed coming from the RT's viewpoint. In return the RT interactively receives annotations and audio / text to the AR glasses **8** themselves in order to guide them through the set-up procedure.

At an operation **308**, once the QA set-up is deemed acceptable, a set of measurements are executed. These measurements run through a set of algorithms employed on the medical imaging device **2.** In some QA procedures, the measurements involve acquiring one or more images of the imaging phantom using the medical imaging device **2.** The measurements may be the acquired images *per se,* or the measurements may be information extracted from the acquired images by post-acquisition image processing. For example, the measurements might include a measurement of a spatial resolution obtained by the acquired images, or a measurement of pixel intensities, of image contrast metrics, and/or so forth. Where the measurements involve post-acquisition image processing, they may be done automatically by the controller of the medical imaging device **2** or manually (or semi-manually) by the RT.

At an operation **310**, a verification of phantom placement is performed to assess the quality metrics obtained in operation **308.** In some examples, the CNN **48** can analyze the quality metrics to learn from new performed procedures. The QA metrics and placement information are presented in the form of textual information, dashboards, and images to the RT (via the ROCC device **8)** and RSE (via the remote workstation **12).**

If adjustments to item placement or measurements are required, then the respective operations **304-310** are repeated as indicated by the flowback arrow in Fig.3. If the phantom placement was correct but the QA metrics are non-optimal as assessed in operation **310**, then the operation **310** may additionally recommend adjustments to the imaging device **2** to improve these metrics. After the RT makes the recommended adjustments, flow may again return to operations **308** and **310** as indicated by the flowback arrow to re-image the phantom and see if the adjustments improved the QA metrics. If (or when, after adjustment) the measurements are deemed successful, then, at an operation **312**, a comprehensive report is auto generated by the ROCC device 8 for the RT and RSE. The report template remains adjustable for the customer to account for various kinds of additional measurements coming due to the supplemental QA phantoms or components required to match the QA metrics. However, the filling of this supplemental fields would be left to the user in case the image analysis algorithms are not capable of extracting the relevant data.

In one example, a required adjustment can include adjusting a coil placed on the patient (e.g., a head coil or a neck coil) during an imaging procedure. In another example, a different type of coil can be substituted for a previously-used coil. In another example, a required adjustment can include changing a phantom used during the imaging procedure. In another example, a required adjustment can further include using one or more pencil dosimeters placed in an acrylic phantom to be used in CT dose index (CTDI) dose measurements. The placement of the dosimeter(s) can be verified by a camera or imaging data acquired by the medical imaging device **120.** These are merely examples, and should not be construed as limiting.

The preceding description of the disclosed embodiments is provided to enable any person skilled in the art to practice the concepts described in the present disclosure. As such, the above disclosed subject matter is to be considered illustrative, and not restrictive, and the appended claims are intended to cover all such modifications, enhancements, and other embodiments which fall within the true spirit and scope of the present disclosure. Thus, to the maximum extent allowed by law, the scope of the present disclosure is to be determined by the broadest permissible interpretation of the following claims and their equivalents and shall not be restricted or limited by the foregoing detailed description.

In the foregoing detailed description, for the purposes of explanation and not limitation, representative embodiments disclosing specific details are set forth in order to provide a thorough understanding of an embodiment according to the present teachings. Descriptions of known systems, devices, materials, methods of operation and methods of manufacture may be omitted so as to avoid obscuring the description of the representative embodiments. Nonetheless, systems, devices, materials, and methods that are within the purview of one of ordinary skill in the art are within the scope of the present teachings and may be used in accordance with the representative embodiments. It is to be understood that the terminology used herein is for purposes of describing particular embodiments only and is not intended to be limiting. The defined terms are in addition to the technical and scientific meanings of the defined terms as commonly understood and accepted in the technical field of the present teachings.

It will be understood that, although the terms first, second, third, etc. may be used herein to describe various elements or components, these elements or components should not be limited by these terms. These terms are only used to distinguish one element or component from another element or component. Thus, a first element or component discussed below could be termed a second element or component without departing from the teachings of the inventive concept.

The terminology used herein is for purposes of describing particular embodiments only and is not intended to be limiting. As used in the specification and appended claims, the singular forms of terms "a," "an" and "the" are intended to include both singular and plural forms, unless the context clearly dictates otherwise. Additionally, the terms "comprises," "comprising," and/or similar terms specify the presence of stated features, elements, and/or components, but do not preclude the presence or addition of one or more other features, elements, components, and/or groups thereof. As used herein, the term "and/or" includes any and all combinations of one or more of the associated listed items.

Unless otherwise noted, when an element or component is said to be "connected to," "coupled to," or "adjacent to" another element or component, it will be understood that the element or component can be directly connected or coupled to the other element or component, or intervening elements or components may be present. That is, these and similar terms encompass cases where one or more intermediate elements or components may be employed to connect two elements or components. However, when an element or component is said to be "directly connected" to another element or component, this encompasses only cases where the two elements or components are connected to each other without any intermediate or intervening elements or components.

The present disclosure, through one or more of its various aspects, embodiments and/or specific features or sub-components, is thus intended to bring out one or more of the advantages as specifically noted below. For purposes of explanation and not limitation, example embodiments disclosing specific details are set forth in order to provide a thorough understanding of an embodiment according to the present teachings. However, other embodiments consistent with the present disclosure that depart from specific details disclosed herein remain within the scope of the appended claims. Moreover, descriptions of well-known apparatuses and methods may be omitted so as to not obscure the description of the example embodiments. Such methods and apparatuses are within the scope of the present disclosure.

## Claims

1. A non-transitory computer readable medium **(26s)** storing instructions executable by at least one electronic processor **(14s)** to perform a method **(100)** of monitoring a quality assurance (QA) procedure performed using a medical device **(2)**, the method comprising:
receiving a signal indicating a start of the QA procedure;
analyzing video **(17)** of the medical device acquired after receiving the signal to detect one or more errors during the QA procedure; and
providing a remedial action addressing the detected one or more errors.

2. The non-transitory computer readable medium **(26s)** of claim 1, wherein the analyzing of the video **(17)** includes:
analyzing the video to detect a sequence of events of the QA procedure; and
detecting an error during the QA procedure by comparing the detected sequence of events with a workflow of the QA procedure.

3. The non-transitory computer readable medium **(26s)** of either one of claims 1 and 2, wherein the analyzing of the video (17) includes:
detecting an object being used in the QA procedure that is different from a correct object for the QA procedure.

4. The non-transitory computer readable medium **(26s)** of claim 1, wherein the analyzing of the video **(17)** includes:
detecting an incorrect position of an object used in the QA procedure.

5. The non-transitory computer readable medium **(26s)** of either one of claims 3 and 4, wherein one of:
(i) the medical device (2) comprises a medical imaging device and the object comprises an imaging phantom used in the QA procedure; or
(ii) the medical device comprises a magnetic resonance imaging (MRI) scanner and the object comprises a local coil used in the QA procedure.

6. The non-transitory computer readable medium **(26s)** of any one of claims 2-5, wherein the analyzing includes detecting the object in the video **(17)** using a neural network (NN) **(48)** applied to one or more frames of the video.

7. The non-transitory computer readable medium **(26s)** of any one of claims 1-6, wherein the medical device **(2)** comprises a medical imaging device and the method **(100)** further includes:
receiving an image acquired by the medical imaging device during the QA procedure;
analyzing the received image to identify a deficiency of the medical imaging device;
determining an adjustment to the medical imaging device for resolving the identified deficiency of the medical imaging device; and
proposing the adjustment via a display device **(36).**

8. The non-transitory computer readable medium **(26s)** of any one of claims 1-7, wherein providing a remedial action addressing the detected one or more errors includes:
displaying a visualization **(40)** of the remedial action on a display device **(36)** observable by a person performing the QA procedure.

9. The non-transitory computer readable medium **(26s)** of any one of claims 1-7, wherein providing a remedial action addressing the detected one or more errors includes:
displaying a visualization **(40)** of the remedial action on an augmented reality (AR) display **(8)** worn by a person performing the QA procedure, the visualization being superimposed on the medical device in the AR display.

10. The non-transitory computer readable medium **(26s)** of any one of claims 1-8, wherein providing a remedial action addressing the detected one or more errors includes:
displaying a visualization **(40)** of the remedial action on a display device **(36)** of a projector device **(8)** wearable by a person performing the QA procedure.

11. The non-transitory computer readable medium **(26s)** of any one of claims 1-8, wherein providing a remedial action addressing the detected one or more errors includes:
displaying a visualization **(40)** of the remedial action on a display device **(36)** of a heads-up display (HUD) device **(8)** wearable by a person performing the QA procedure.

12. The non-transitory computer readable medium **(26s)** of any one of claims 1-8, wherein providing a remedial action addressing the detected one or more errors includes:
controlling a position of the medical device **(2).**

13. The non-transitory computer readable medium **(26s)** of any one of claims 1-8, wherein providing a remedial action addressing the detected one or more errors includes:
performing a validation process to determine if the detected one or more errors corresponds to a predetermined matter with the medical device **(2).**

14. The non-transitory computer readable medium **(26s)** of any one of claims 1-8, further storing instructions executable by at least one electronic processor **(14s)** to perform a remote assistance method (200) including:
displaying the video **(17)** of the medical device on a remote expert workstation **(12)** located remotely from the medical device **(2)**; and
providing two-way communication between the remote expert workstation and an electronic device **(8)** disposed with the medical device.

15. The non-transitory computer readable medium **(26s)** of claim 14, wherein the remote assistance method **(200)** further includes:
determine whether an imaging procedure using the medical imaging device **2** is a clinical scan or a QA scan; and
wherein the received signal indicating the start of the QA procedure comprises determination that the imaging procedure is a QA scan.
